# EUROPÄISCHE PATENTANMELDUNG

(11) **EP 2 264 141 A1**
(43) Veröffentlichungstag der Anmeldung: **22.12.2010**
(21) Anmeldenummer: 09007828.8
(22) Anmeldetag: 15.06.2009
(51) Int. Cl.: C12M 1/107

(54) **Verfahren und Vorrichtung zur Erzeugung von Biogas**

(71) Anmelder: KOMPOFERM GmbH, 33428 Marienfeld (DE)
(72) Erfinder: Eggersmann, Karlgünter, 33428 Marienfeld (DE)
(74) Vertreter: Schober, Mirko

(57) **Zusammenfassung**

Bei einer Vorrichtung zur Durchführung eines Verfahrens zur Biogasgewinnung sind wenigstens eine Nassfermentationseinrichtung (1) und wenigstens eine Trockenfermentationseinrichtung (2) vorgesehen. Es ist eine Trenneinrichtung (3) vorgesehen, welche dazu ausgelegt ist, ein in der Vorrichtung zur Biogasgewinnung einzusetzendes Substratmaterial in wenigstens zwei Fraktionen (31, 32) zur Beschickung eines Nassfermenters (1c) und eines Trockenfermenters (2a) aufzuteilen.

## Beschreibung

Die Erfindung betrifft ein Verfahren zur Erzeugung von Biogas nach dem Oberbegriff des Anspruchs 1.

Bei entsprechenden Verfahren wird mit Hilfe eines Fermentationsprozesses aus Biomasse methanhaltiges Biogas gewonnen. Bei den bekannten Verfahren müssen jedoch bestimmte Anforderungen an die Auswahl der als Substrat zu verwendenden Biomasse gestellt werden. Für die sog. Nassfermentation bedarf es der Auflösung/Suspension des Substrates in einer Flüssigkeit, damit die Biogasgewinnung in einem Flüssigfermenter gut funktioniert. Bei der Trockenfermentation, bei der das Substrat perkoliert - d.h. mit einem Perkolat durchströmt - wird, soll eine entsprechende Auflösung nach Möglichkeit nicht stattfinden.

Dies führt dazu, dass je nach Art der vorhandenen Anlagentechnik nur bestimmte Substrate für die unterschiedlichen Prozesse eingesetzt werden können.

Es ist daher Aufgabe der vorliegenden Erfindung, ein Verfahren und eine Vorrichtung der eingangs genannten Art zu schaffen, mit denen aus Substrat mit einer sehr großen Bandbreite an Teilchengrößen Biogas gewonnen werden kann.

Gelöst wird diese Aufgabe durch ein Verfahren mit den Merkmalen des Anspruchs 1 sowie eine Vorrichtung mit den Merkmalen des Anspruchs 8. Vorteilhafte Ausführungsformen finden sich in den abhängigen Ansprüchen.

Die Erfindung trennt vor der Fermentation die zugeführte Biomasse in wenigstens zwei Teilfraktionen auf, von welchen der Beschaffenheit nach die erste einer Nassfermentation und die zweite einer Trockenfermentation zugeführt werden kann. Die Trennung erfolgt bevorzugt über eine Selektion nach der Teilchengröße der in der zugeführten Biomasse enthaltenen Teilchen, so dass für die Nassfermentation eine organische Feinfraktion und für Trockenfermentation eine organische Grobfraktion als jeweiliges Substrat zur Verfügung steht. Die Trennung in Teilfraktionen kann auch auf andere Weise (z.B. durch Selektion nach der Dichte der Teilchen) erfolgen.

Nach einer bevorzugten Ausführungsform sind die Nassfermentation und die Trockenfermentation verschachtelt ausgebildet, so dass aus der Nassfermentation entstehendes Brauch-oder Prozesswasser der Trockenfermentation zugeführt und aus der Trockenfermentation aufgefangenes Perkolat der Nassfermentation zugeführt werden. Bevorzugt kann ein Nassfermenter gleichzeitig als Perkolatbehälter der Trockenfermentation eingesetzt werden.

Die Erfindung wird nachfolgend anhand der in den Figuren 1 bis 3 gezeigten Ausführungsbeispiele schematisch näher erläutert.
- Figur 1 -: zeigt eine Prinzipskizze einer ersten erfin- dungsgemäßen Ausführungsform,
- Figur 2 -: zeigt eine Prinzipskizze einer zweiten erfin- dungsgemäßen Ausführungsform,
- Figur 3 -: zeigt eine Prinzipskizze einer dritten erfin- dungsgemäßen Ausführungsform.

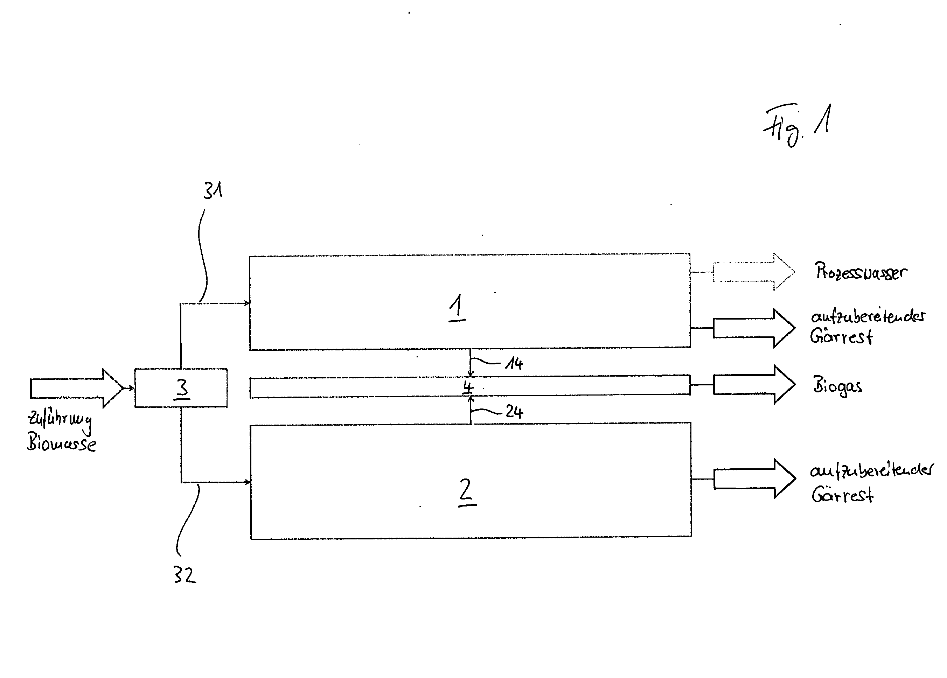

In Figur 1 ist das erfindungsgemäße Prinzip dargestellt. Dabei sind wenigstens eine Nassfermentationseinrichtung 1 und eine Trockenfermentationseinrichtung 2 vorgesehen, die bevorzugt so geschaltet sind, dass das in ihnen hergestellte Biogas über Leitungen 14 bzw. 24 in ein gemeinsames Biogassystem 4 geführt und von dort aus in bekannter Weise zur weiteren Verwendung befördert wird. Aus der Nassfermentation entsteht zudem zum einen Prozesswasser, welches abzuführen ist, zum anderen ein ggf. aufzubereitender Gärrest. Aus der Trockenfermentationseinrichtung 2 entsteht neben dem Biogas ebenfalls ein Gärrest. Für den Betrieb eines Fermenters ist ein Substrat notwendig, welches beispielsweise aus Biomasse wie einem organischen Abfallstrom gewonnen wird. Erfindungsgemäß ist den Fermentationseinrichtungen 1, 2 eine Trenneinrichtung 3 vorgeschaltet, welche zugeführte Biomasse ihrer späteren Verwendung entsprechend vorselektiert und wenigstens eine erste Fraktion 31 zur Beschickung eines Nassfermenters und eine zweite Fraktion 32 zur Beschickung eines Trockenfermenters aus der Ausgangsmasse generiert. Dies kann zum Bespiel durch Aufteilen der Ströme nach Größe der Teilchen in der Ausgangsmasse geschehen oder durch eine Dichteselektion. In jedem Fall kann durch diese Maßnahme ein weitaus größerer Nutzbereich verschiedender Partikelgrößen direkt zur Gewinnung von Biogas eingesetzt werden als bei üblichen Anlagen.

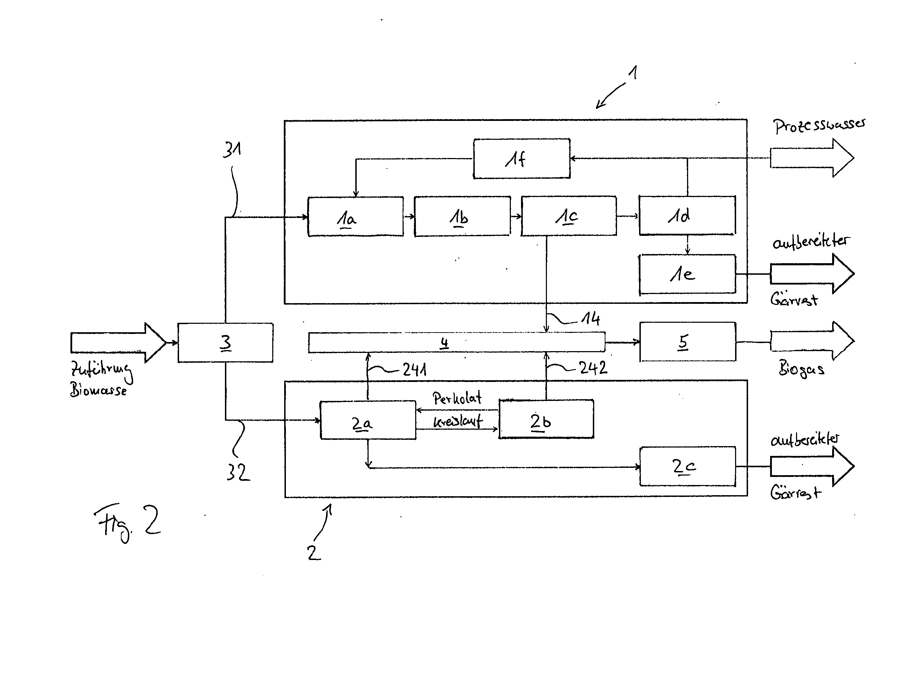

In Figur 2 ist eine mögliche Variante dargestellt, wobei bei der Nassfermentation 1 und der Trockenfermentation 2 die einzelnen Prozessschritte als Blockschaltbild genauer gezeigt sind.

Bei der Nassfermentation 1 wird die Fraktion 31, bevorzugt eine organikreiche Fraktion aus feineren Teilen der Ausgangsmasse, zunächst unter Zugabe von Brauchwasser aufgelöst (1a), die Lösung wird anschließend hydrolysiert (1b) und in einen Flüssig- oder Nassfermenter 1c verbracht, in welchem durch Fermentation Biogas entsteht, welches über den Auslass 14 in das Biogassystem 4 gelangt. Die im Flüssigfermenter enthaltene Suspension wird in einem anschlie-Qenden Schritt 1d in eine feste Phase und eine flüssige Phase aufgetrennt. Die feste Phase bildet den Gärrest, der in einer internen oder externen Aufbereitung 1e aufbereitet werden kann. Die Flüssigphase wird teils als Prozesswasser abgeleitet und teils - bevorzugt über einen Brauchwasserspeicher 1f - als Brauchwasser dem Prozess zur Stoffauflösung 1a oder an anderer Stelle wieder zugeführt.

Bei der Trockenfermentation 2 gelangt die Fraktion 32, bevorzugt eine organikreiche Fraktion aus gröberen Teilen der Ausgangsmasse, als Substrat in den Trockenfermenter 2a und wird mit Perkolat aus einem Perkolatspeicher 2b im Kreislaufverfahren perkoliert. Dabei entsteht Biogas im Trockenfermenter 2a, welches über die Leitung 241 dem Biogassystem 4 zugeführt wird. Im Perkolatspeicher 2b entsteht ebenfalls Biogas, welches ebenso dem Biogassystem 4 über die Leitung 242 zugeführt wird. Die übrig bleibende feste Phase kann als Gärrest in einer internen oder externen Aufbereitung 2c aufbereitet und beispielsweise dem Prozess zum Animpfen des Substrates wieder zugeführt werden. Die Gärrestaufbereitungen 1e und 2c können extern zusammengelegt werden, so dass lediglich eine einzige Aufbereitungsstufe gebildet wird.

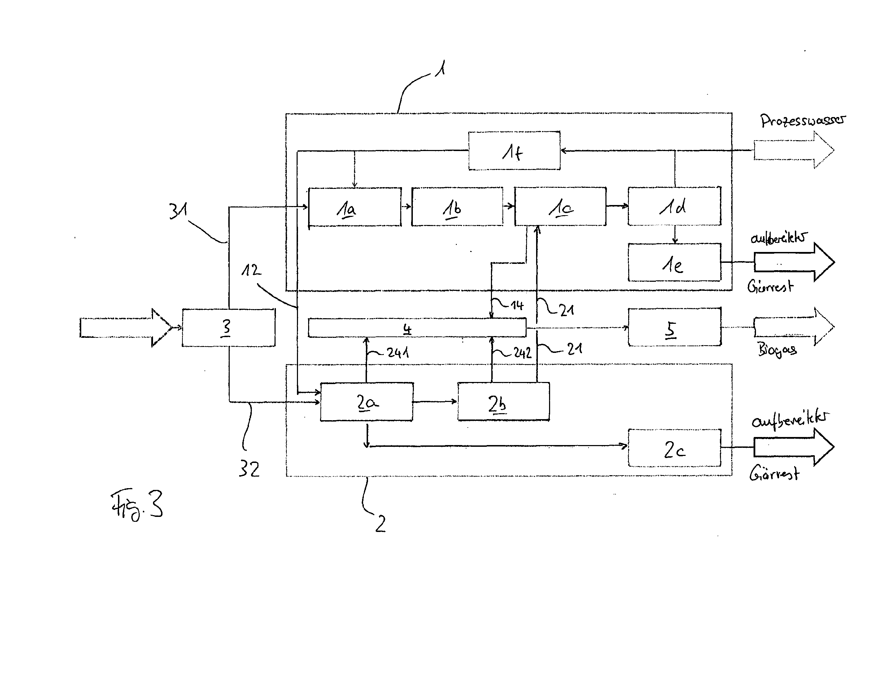

Eine modifizierte Ausführungsform ist in der Figur 3 dargestellt. Zu Vermeidung von Wiederholungen wird auf die obigen Ausführungen verwiesen. Im Unterschied zur in Figur 2 gezeigten Variante wird das aus der Nassfermentation 1 gewonnene Prozess- oder Brauchwasser nicht dem Nass-, sondern dem Trockenfermentationsprozess 2 über die Verbindungsleitung 12 zur Verfügung gestellt und dort als Perkolat in den Trockenfermenter 2a eingeleitet. Das im Trockenfermenter aufgefangene Perkolat wird sodann entweder in einen (jetzt optionalen) Perkolatbehälter 2b oder direkt in den Nassfermenter 1c über eine weitere Verbindungsleitung 21 eingeleitet. Bei der letzteren Alternative (die Leitung 242 entfällt in diesem Fall) kann der Nassfermenter 1c als Perkolatbehälter des Trockenfermentationsprozesses betrieben werden. Durch diesen verschachtelten Prozess können beide Fermentationsverfahren synergetisch betrieben werden, so dass auch der apparative Aufwand verringert werden kann.

### Bezugszeichenliste:

- 1: Nassfermentation
- 1a: Stoffauflösung
- 1b: Hydrolyse
- 1c: Flüssigfermenter
- 1d: Fest-/Flüssigtrennung
- 1e: Gärrestnachbehandlung
- 1f: Brauchwasserspeicher
- 2: Trockenfermentation
- 2a: Trockenfermenter
- 2b: Perkolatbehälter
- 2c: Gärrestnachbehandlung
- 3: Trenneinrichtung
- 4: Biogassystem
- 5: Biogasaufbereitung
- 12: Verbindungsleitung Nassfermentation/ Trockenfermentation
- 14: Verbindungsleitung Nassfermentation/Biogassystem
- 21: Verbindungsleitung Trockenfermentation/ Nassfermentation
- 24, 241, 242:: Verbindungsleitungen Trockenfermentation/ Biogassystem
- 31: erste Fraktion nach Trennung
- 32: zweite Fraktion nach Trennung

## Patentansprüche

1. Verfahren zur Erzeugung von Biogas durch Fermentation eines Substratmaterials, bei welchem das Substratmaterial wenigstens einem Fermenter zugeführt wird, **dadurch gekennzeichnet,**
**dass** vor dem Zuführen des Substratmaterials zum Fermenter (1c; 2a) das Substrat in wenigstens zwei Fraktionen aufgeteilt wird, welche anschließend jeweils einem Fermenter zugeführt werden.

2. Verfahren nach Anspruch 1,
**dadurch gekennzeichnet,**
**dass** wenigstens eine Fraktion nach dem Aufteilen einem Nassfermentationsprozess (1) und wenigstens eine weitere Fraktion nach dem Aufteilen einem Trockenfermentationsprozess (2) zugeführt werden.

3. Verfahren nach einem der Ansprüche 1 oder 2,
**dadurch gekennzeichnet,**
**dass** die Aufteilung des Substratmaterials anhand der Teilchengröße oder der Dichte der im Substrat enthaltenen Teilchen erfolgt.

4. Verfahren nach einem der Ansprüche 2 oder 3,
**dadurch gekennzeichnet,**
**dass** wenigstens ein Flüssigfermenter (1c) und wenigstens ein Trockenfermenter (2a) eingesetzt werden, in denen Biogas erzeugt wird, wobei hinter dem Flüssigfermenter (1c) eine Trennung von fester und flüssiger Phase erfolgt und die Flüssigphase wenigstens teilweise dem Trocken- und oder Nassfermentationsprozess wieder zugeführt wird.

5. Verfahren nach einem der Ansprüche 2 bis 4,
**dadurch gekennzeichnet,**
**dass** der Flüssigfermenter (1c) gleichzeitig als Perkolatbehälter des Trockenfermentationsprozesses betrieben wird.

6. Verfahren nach einem der Ansprüche 4 oder 5,
**dadurch gekennzeichnet,**
**dass** eine Gärrestnachbehandlung erfolgt.

7. Verfahren nach Anspruch 6,
**dadurch gekennzeichnet,**
**dass** Gärrest dem Fermentationsprozess teilweise wieder zugeführt wird.

8. Vorrichtung zur Durchführung eines Verfahrens zur Biogasgewinnung, insbesondere nach einem der vorherigen Ansprüche,
**dadurch gekennzeichnet,**
**dass** wenigstens eine Nassfermentationseinrichtung (1) und wenigstens eine Trockenfermentationseinrichtung (2) vorgesehen sind, und dass eine Trenneinrichtung (3) vorgesehen ist, welche dazu ausgelegt ist, ein in der Vorrichtung zur Biogasgewinnung einzusetzendes Substratmaterial in wenigstens zwei Fraktionen (31, 32) zur Beschickung eines Nassfermenters (1c) und eines Trockenfermenters (2a) aufzuteilen.

9. Vorrichtung nach Anspruch 8,
**dadurch gekennzeichnet,**
**dass** eine Verbindungseinrichtung (12) vorgesehen ist, die dazu ausgelegt ist, eine aus der Nassfermentationseinrichtung (1) gewonnene Flüssigphase wenigstens teilweise der Trockenfermentationseinrichtung (2) zuzuführen.

10. Vorrichtung nach Anspruch 9,
**dadurch gekennzeichnet,**
**dass** eine weitere Verbindungseinrichtung (21) vorgesehen ist, welche dazu ausgelegt ist, aus der Trockenfermentationseinrichtung (2) entstammendes Perkolat der Nassfermentationseinrichtung (1) zuzuführen.

## Geänderte Patentansprüche

### Geänderte Patentansprüche gemäss Regel 137(2) EPÜ.

**1.** Verfahren zur Erzeugung von Biogas durch Fermentation eines Substratmaterials, bei welchem das Substratmaterial wenigstens einem Fermenter zugeführt wird,
**dadurch gekennzeichnet,**
**dass** vor dem Zuführen des Substratmaterials zum Fermenter (1c; 2a) das Substrat in wenigstens zwei Fraktionen aufgeteilt wird, welche anschließend jeweils einem Fermenter zugeführt werden, wobei wenigstens eine Fraktion nach dem Aufteilen einem Nassfermentationsprozess (1) und wenigstens eine weitere Fraktion nach dem Aufteilen einem Trockenfermentationsprozess (2) zugeführt werden.

**2.** Verfahren nach Anspruch 1,
**dadurch gekennzeichnet,**
**dass** die Aufteilung des Substratmaterials anhand der Teilchengröße oder der Dichte der im Substrat enthaltenen Teilchen erfolgt.

**3.** Verfahren nach Anspruch 2,
**dadurch gekennzeichnet,**
**dass** wenigstens ein Flüssigfermenter (1c) und wenigstens ein Trockenfermenter (2a) eingesetzt werden, in denen Biogas erzeugt wird, wobei hinter dem Flüssigfermenter (1c) eine Trennung von fester und flüssiger Phase erfolgt und die Flüssigphase wenigstens teilweise dem Trocken- und oder Nassfermentationsprozess wieder zugeführt wird.

**4.** Verfahren nach einem der Ansprüche 2 bis 3,
**dadurch gekennzeichnet,**
**dass** der Flüssigfermenter (1c) gleichzeitig als Perkolatbehälter des Trockenfermentationsprozesses betrieben wird.

**5.** Verfahren nach einem der Ansprüche 3 oder 4,
**dadurch gekennzeichnet,**
**dass** eine Gärrestnachbehandlung erfolgt.

**6.** Verfahren nach Anspruch 5,
**dadurch gekennzeichnet,**
**dass** Gärrest dem Fermentationsprozess teilweise wieder zugeführt wird.

**7.** Vorrichtung zur Durchführung eines Verfahrens zur Biogasgewinnung, insbesondere nach einem der vorherigen Ansprüche,
**dadurch gekennzeichnet,**
**dass** wenigstens eine Nassfermentationseinrichtung (1) und wenigstens eine Trockenfermentationseinrichtung (2) vorgesehen sind, und dass eine Trenneinrichtung (3) vorgesehen ist, welche dazu ausgelegt ist, ein in der Vorrichtung zur Biogasgewinnung einzusetzendes Substratmaterial in wenigstens zwei Fraktionen (31, 32) zur Beschickung eines Nassfermenters (1c) und eines Trockenfermenters (2a) aufzuteilen.

**8.** Vorrichtung nach Anspruch 7,
**dadurch gekennzeichnet,**
**dass** eine Verbindungseinrichtung (12) vorgesehen ist, die dazu ausgelegt ist, eine aus der Nassfermentationseinrichtung (1) gewonnene Flüssigphase wenigstens teilweise der Trockenfermentationseinrichtung (2) zuzuführen.

**9.** Vorrichtung nach Anspruch 8,
**dadurch gekennzeichnet,**
**dass** eine weitere Verbindungseinrichtung (21) vorgesehen ist, welche dazu ausgelegt ist, aus der Trockenfermentationseinrichtung (2) entstammendes Perkolat der Nassfermentationseinrichtung (1) zuzuführen.
